(19) **Europäisches Patentamt / European Patent Office / Office européen des brevets**

(11) **EP 1 558 658 B1**

(12) # FASCICULE DE BREVET EUROPEEN

(45) Date de publication et mention de la délivrance du brevet:
**19.12.2012 Bulletin 2012/51**

(21) Numéro de dépôt: **03767870.3**

(22) Date de dépôt: **31.10.2003**

(51) Int Cl.:
*C08F 293/00* (2006.01)    *C11D 1/88* (2006.01)
*C11D 3/37* (2006.01)

(86) Numéro de dépôt international:
**PCT/FR2003/003255**

(87) Numéro de publication internationale:
**WO 2004/044023 (27.05.2004 Gazette 2004/22)**

(54) **COPOLYMERE A STRUCTURE CONTROLEE PRESENTANT UNE PARTIE AMPHOTERE OU ZWITTERIONIQUE.**

COPOLYMER MIT KONTROLLIERTER STRUKTUR MIT EINEM AMPHOTEREN ODER ZWITTERIONISCHEN TEIL

CONTROLLED STRUCTURE COPOLYMER COMPRISING AN AMPHOTERIC OR ZWITTERIONIC PART

(84) Etats contractants désignés:
**AT BE BG CH CY CZ DE DK EE ES FI FR GB GR HU IE IT LI LU MC NL PT RO SE SI SK TR**

(30) Priorité: **07.11.2002 FR 0213950**
**29.08.2003 FR 0310292**

(43) Date de publication de la demande:
**03.08.2005 Bulletin 2005/31**

(73) Titulaire: **RHODIA CHIMIE**
**92512 Boulogne Billancourt Cedex (FR)**

(72) Inventeur: **DESTARAC, Mathias**
**F-75005 Paris (FR)**

(74) Mandataire: **Boittiaux, Vincent et al**
**Rhodia Services**
**Direction de la Propriété Industrielle**
**40, rue de la Haie-Coq**
**93306 Aubervilliers Cedex (FR)**

(56) Documents cités:
**WO-A-02/28929**        **WO-A-99/35177**
**FR-A- 2 794 464**        **US-A- 5 219 945**
**US-A- 6 074 628**        **US-A1- 2002 143 127**
**US-A1- 2003 105 201**

**Description**

La présente invention a pour objet une nouvelle famille de polymères. On connaît de nombreuses structures de polymères et de nombreuses compositions. Il existe par exemple des homopolymères, des copolymères linéaires, statistiques ou séquencés (copolymères à blocs), des polymères ou copolymères peignes ou greffés, des polymères ou copolymères étoiles. La composition d'un copolymère est liée aux différentes unités comprises dans les chaînes polymériques. Les différentes unités peuvent dériver de différents monomères et/ou résulter d'un réaction chimique mise en oeuvre après le processus de polymérisation. Parmi les copolymères on connaît des copolymères statistiques comprenant des unités hydrophobes neutres, des unités anioniques ou potentiellement anioniques, et des unités cationiques ou potentiellement cationiques. On précise que les copolymères statistiques sont généralement obtenus en introduisant ensemble différents monomères dans un milieu réactionnel de polymérisation. De tels copolymères sont des polymères à structure non contrôlée. Ces polymères présentent des propriétés intéressantes, qui peuvent les rendre utiles dans des compositions telles que des compositions détergentes, pour jouer un rôle de dépôt sur des surfaces, ou pour déclencher de processus de précipitation d'autres composés par variation de conditions extérieures comme le pH.

Récemment des procédés de préparation de polymères à structure contrôlée ont été développés, notamment pour l'obtention de copolymères à blocs. Ainsi, on a trouvé qu'il existe des procédés et des conditions opératoires permettant de préparer des copolymères à blocs présentant deux blocs, de type (neutre hydrophile)-bloc-(anionique hydrophile), (neutre hydrophile)-bloc-(cationique hydrophile), (neutre hydrophobe)-bloc-(neutre hydrophile), (neutre hydrophobe)-bloc-(anionique hydrophile), (neutre hydrophobe)-bloc-(cationique hydrophile). Ces différentes familles de polymères s'avèrent utiles dans certaines compositions.

WO 02/28929 décrit des copolymères à structure contrôlée, comprenant 2 unités A et B de composition différente, l'une de ces unités étant formée par un monomère insaturé comprenant un monomère zwitterionique d'une certaine formule YBX', l'autre pouvant être de même nature ou différentes. Ces copolymères témoignent une biocompatibilité améliorée et sont utilisés dans les systèmes d'administration de produits médicamenteux.

Toutefois, on cherche toujours de nouveau polymères pour obtenir de nouvelles compositions ou des nouveaux systèmes physico-chimiques.

Ainsi, l'invention propose un copolymère à structure contrôlée comprenant au moins deux parties distinctes, une première partie A amphotère comprenant des unités anioniques ou potentiellement anioniques, et des unités cationiques ou potentiellement cationiques, et une autre partie B, non amphotère ou zwitterionique. Il présente en outre de grands potentiels d'adaptation, par variation de sa composition, afin d'améliorer ou de modifier les propriétés de compositions dans lesquels il est introduit, ou plus simplement afin de proposer de nouvelles compositions ou systèmes physico-chimiques. En d'autres termes, l'invention propose un nouveau copolymère présentant une grande modularité. On peut ainsi moduler les propriétés d'un copolymère amphotère statistique par ajout d'un bloc, par exemple un bloc neutre, ou coupler les propriétés de plusieurs séquences, dont une séquence amphotère.

Le copolymère selon l'invention trouve des utilisations dans de nombreux domaines, notamment dans les domaines de la détergence, du soin du linge, de la cosmétique, et des compositions destinées à nettoyer, traiter ou protéger la peau ou les cheveux. L'invention concerne donc également l'utilisation du copolymère dans des compositions de détergence, de soin du linge, ou de nettoyage, traitement, et/ou protection de la peau et/ou des cheveux.

Le copolymère à structure contrôlée, comprend au moins deux parties A et B de compositions distinctes, la partie A comprenant des unités ioniques ou potentiellement ioniques, caractérisé en ce que:

- la partie A est une partie amphotère comprenant:

    - des unités $A_C$ cationiques ou potentiellement cationiques,
    - des unités $A_A$ anioniques ou potentiellement anioniques, et
    - optionnellement des unités $A_N$ neutres, hydrophiles et/ou hydrophobes,

- la partie B n'est pas une partie amphotère ou zwitterionique.

Les parties d'un copolymère à structure contrôlée peuvent être notamment des blocs, des squelettes linéaires, des chaînes latérales, des greffons, des «cheveux» ou branches de microgels ou d'étoiles, des coeurs d'étoiles ou de microgels, ou bien encore des parties de chaînes polymériques présentant différentes concentrations en différentes unités.

Ainsi la structure contrôlée, que présente le copolymère selon l'invention, peut être choisie parmi les structures suivantes:

- copolymère à blocs, comprenant au moins deux blocs, la partie A correspondant à un bloc, la partie B correspondant à un autre. La partie A est le plus souvent constituée de plusieurs unités différentes, présentant le cas échéant un gradient de composition. La partie A peut également présenter une structure de copolymère statistique. Ainsi la partie A peut présenter une structure copolymère statistique ou copolymère à gradient de composition.

- copolymère peigne ou greffé, comprenant un squelette et des chaînes latérales, avec la partie A correspondant au squelette et la partie B correspondant à des chaînes latérales, ou avec la partie B correspondant au squelette et la partie A correspondant à des chaînes latérales.

- copolymère étoile ou microgel, comprenant un coeur polymérique ou non polymérique, et des chaînes polymériques périphériques, une partie correspondant au coeur, l'autre correspondant aux chaînes périphériques. La partie A peut correspondre au coeur et la partie B peut correspondre aux chaînes périphériques. Inversement, la partie B peut correspondre au coeur et la partie A peut correspondre aux chaînes périphériques.

Selon un mode de réalisation particulièrement intéressant, le copolymère est un copolymère à blocs, par exemple un copolymère dibloc (bloc A)-(bloc B), tribloc (bloc A)-(bloc B)-(bloc A) ou tribloc (bloc B)-(bloc A)-(bloc B).

Définitions

Dans la présente demande, on désigne par unité dérivant d'un monomère une unité qui peut être obtenue directement à partir dudit monomère par polymérisation. Ainsi, par exemple, une unité dérivant d'un ester d'acide acrylique ou méthacrylique ne couvre pas une unité de formule —$CH_2$-CH(COOH)-, —$CH_2$-C($CH_3$)(COOH)-, —$CH_2$-CH (OH)-, respectivement, obtenue par exemple en polymérisant un ester d'acide acrylique ou méthacrylique, ou de l'acétate de vinyle, respectivement, puis en hydrolysant. Une unité dérivant d'acide acrylique ou méthacrylique couvre par exemple une unité obtenue en polymérisant un monomère (par exemple un ester d'acide acrylique ou méthacrylique), puis en faisant réagir (par exemple par hydrolyse) le polymère obtenu de manière à obtenir des unités de formule —$CH_2$-CH (COOH)-, ou - $CH_2$-C($CH_3$)(COOH)-. Une unité dérivant d'un alcool vinylique couvre par exemple une unité obtenue en polymérisant un monomère (par exemple un ester vinylique), puis en faisant réagir (par exemple par hydrolyse) le polymère obtenue de manière à obtenir des unités de formule —$CH_2$-CH(OH)-.

Dans la présente demande, sauf mention contraire, les masses molaires moyennes sont des masses molaires moyennes en nombre, mesurées par chromatographie d'exclusion stérique dans un solvant approprié, couplée à un détecteur de diffusion de lumière multi-angle (GPC-MALLS). Dans la présente demande, on peut aussi se référer à des masses molaires moyennes théoriques, déterminées à partir des masses de constituants utilisés pour préparer les polymères.

Typiquement, la masse molaire moyenne théorique M d'un bloc, d'une chaîne latérale, d'un squelette, de chaînes périphériques, ou d'un coeur est calculée selon la formule suivante:

$$M = \sum_i M_i * \frac{n_i}{n_{precursor}},$$

où $M_i$ est la masse molaire d'un monomère i, $n_i$ est le nombre de moles du monomère i, $n_{precursor}$ est le nombre de moles d'un composé auquel sera liée la chaîne macromoléculaire du bloc, chaîne latérale, squelette, chaîne périphérique, ou coeur. Ce composé peut être un agent de transfert (ou un groupe de transfert) ou un amorceur, un bloc précédent etc. S'il s'agit d'un bloc précédent, le nombre de moles peut être considéré comme le nombre de moles d'un composé auquel la chaîne macromoléculaire dudit bloc précédent a été liée, par exemple un agent de transfert (ou un groupe de transfert) ou un amorceur.

Dans la présente demande, on désigne par une charge moyenne Q d'une partie, la charge définie par l'équation suivante:

$$Q = \frac{[c]X_c - [a]X_a}{[c]X_c + [a]X_a}$$

où:

- [c] est la concentration molaire en unités $A_C$ dans la partie A,
- [a] est la concentration molaire en unités $A_A$ dans la partie A,
- $X_C$ représente le taux de neutralisation éventuelle des unités $A_C$ (dans le cas ou les unités $A_C$ sont potentiellement cationiques); $X_C$ = [BH+]/([B] + [BH+]),
- $X_A$ représente le taux de neutralisation éventuelle des unités $A_A$ (dans le cas ou les unités $A_C$ sont potentiellement anioniques); $X_A$ = [A]/([AH] + [A⁻]).

Dans la présente demande, le terme «hydrophobe» est utilisé dans son sens usuel de «qui n'a pas d'affinité pour l'eau»; cela signifie que le polymère organique dont il est constitué, pris seul (de même composition et de même masse molaire), formerait une solution macroscopique diphasique dans de l'eau distillée à 25°C, à une concentration supérieure à 1% en poids.

Dans la présente demande, le terme «hydrophile» est également utilisé dans son sens usuel de «qui a de l'affinité pour l'eau», c'est-à-dire n'est pas susceptible de former une solution macroscopique diphasique dans de l'eau distillée à 25°C à une concentration supérieure à 1 % en poids.

Selon un mode de réalisation particulièrement intéressant, le rapport pondéral entre la partie B et la partie A est de 0,01 à 100, par exemple de 0.01 à 1, de préférence de 0,1 à 1 ou de 1 à 100 de préférence de 1 à 10. Il s'agit d'un rapport pondéral entre les quantités de monomères mises en oeuvre pour obtenir le copolymère. Ce rapport est aussi le rapport des masses molaires moyennes théoriques.

Selon un mode de réalisation intéressant, le copolymère est hydrosoluble soluble ou hydrodispersable. Cela signifie que ledit copolymère ne forme pas dans l'eau, sur au moins dans un certain domaine de pH et de concentration, une composition diphasique dans les conditions de mise en oeuvre.

D'une manière préférentielle, les parties A et B dérivent de monomères ethylèniquement insaturés.

Le copolymère selon l'invention peut être présenté notamment sous forme de poudre, sous forme de dispersion dans un liquide ou sous forme de solution dans un solvant (eau ou autre). La forme dépend généralement des exigences liées à l'utilisation du copolymère. Elle peut être aussi liée au procédé de préparation du copolymère.

De préférence, les unités des parties A et B, dérivent de monomères ethylèniquement insaturés, plus préférablement $\alpha$-$\beta$ monoethylèniquement insaturés.

<u>Partie B</u>

La partie B (par exemple le bloc B) est une partie polymérique ne correspondant pas à une partie amphotère ou zwitterionique. En d'autres termes, la partie B ne comprend pas d'unités dérivant de monomère zwitterioniques, ou ne comprend pas simultanément des unités cationiques ou potentiellement cationique et des unités anioniques ou potentiellement anioniques. La partie B est de préférence une partie neutre, hydrophile ou hydrophobe, comprenant des unités dérivant de monomères neutres, hydrophiles ou hydrophobes. Les copolymères dont la partie B est une partie hydrophobe neutre sont particulièrement intéressants. D'une manière préférentielle, la partie B est essentiellement non-ionique ou non-ionisable au pH d'utilisation du copolymère. Tout préférentiellement la partie B est non-ionique. Par exemple la partie B (par exemple le bloc B), dérive d'au moins un monomère non-ionique hydrophobe.

La partie B peut en outre contenir des unités non-ioniques hydrophiles dérivées d'au moins un monomère non-ionique hydrophile, en quantité suffisamment faible pour conserver au bloc un caractère hydrophobe; cette quantité peut aller jusqu'à 10% molaire de l'ensemble des monomères dont dérive ladite partie B (par exemple bloc B).

De même, la partie B peut en outre contenir des unités ioniques ou potentiellement ioniques (notamment cationiques ou potentiellement cationiques) dérivées d'au moins un monomère ionique ou potentiellement ionique (notamment cationique ou potentiellement cationique), ce en quantité mineure, ce afin que ledit polymère conserve son caractère hydrophobe et essentiellement non-ionique; cette quantité peut aller jusqu'à 10% molaire de l'ensemble des monomères dont dérive ladite partie B (par exemple bloc B).

A titre d'exemples de <u>monomères non-ioniques hydrophobes</u> dont peut dériver la partie B (par exemple bloc B), on peut mentionner :

- ● les monomères vinylaromatiques tels que styrène, alpha-méthylstyrène, vinyltoluène...
- ● les halogénures de vinyle ou de vinylidène, comme le chlorure de vinyle, chlorure de vinylidène
- ● les $C_1$-$C_{12}$ alkylesters d'acides $\alpha$-$\beta$ monoéthyléniquement insaturés tels que les acrylates et méthacrylates de méthyle, éthyle, butyle, acrylate de 2-éthylhexyle ...
- ● les esters de vinyle ou d'allyle d'acides carboxyliques saturés tels que les acétates, propionates, versatates, stéarates ... de vinyle ou d'allyle
- ● les nitriles $\alpha$-$\beta$ monoéthyléniquement insaturés contenant de 3 à 12 atomes de carbone, comme l'acrylonitrile, le methacrylonitrile ...
- ● les $\alpha$-oléfines comme l'éthylène ...
- ● les diènes conjugués, comme le butadiène, l'isoprène, le chloroprène,
- ● les monomères susceptibles de générer des chaînes polydiméthylsiloxane (PDMS).

Ainsi la partie B peut être un silicone, par exemple une chaîne polydiméthylsiloxane ou un copolymère comprenant des unités diméthylsiloxy.

A titre d'exemples de <u>monomères hydrophiles non-ioniques éventuels</u>, on peut mentionner :

● les hydroxyalkylesters d'acides $\alpha$-$\beta$ éthyléniquement insaturés comme les acrylates et méthacrylates d'hydroxyéthyle, d'hydroxypropyle, le glycérol monométhacrylate...

● les amides $\alpha$-$\beta$ éthyléniquement insaturés comme l'acrylamide, le N,N-diméthyl méthacrylamide, le N-méthylolacrylamide ...

● les monomères $\alpha$-$\beta$ éthyléniquement insaturés portant un segment polyoxyalkyléné hydrosoluble du type polyoxyde d'éthylène, comme les polyoxyde d'éthylène $\alpha$-méthacrylates (BISOMER S20W, S10W, ... de LAPORTE) ou $\alpha$,$\omega$-diméthacrylates, le SIPOMER BEM de RHODIA (méthacrylate de polyoxyéthylène $\omega$-béhényle), le SIPOMER SEM-25 de RHODIA (méthacrylate de polyoxyéthylène $\omega$-tristyrylphényle) ...

● les monomères $\alpha$-$\beta$ éthyléniquement insaturés précurseurs d'unités ou de segments hydrophiles tels que l'acétate de vinyle qui, une fois polymérisés, peuvent être hydrolysés pour engendrer des unités alcool vinylique ou des segments alcool polyvinylique

● les vinylpyrrolidones

● les monomères $\alpha$-$\beta$ éthyléniquement insaturés de type uréido et en particulier le méthacrylamido de 2-imidazolidinone éthyle (Sipomer WAM II de RHODIA).

Des exemples de monomères ioniques ou potentiellement ioniques pouvant être mis en oeuvre en quantité mineure sont mentionnés plus loin (concernant la partie A).

La masse moléculaire moyenne de la partie B (par exemple bloc B), peut aller de 500 à 100000, de préférence de 500 à 25000 g/mol, mesurée par chromatographie d'exclusion stérique.

<u>Partie A</u>

La partie A comprend des unités ioniques ou potentiellement ioniques. Il s'agit d'une partie amphotère comprenant:

- des unités $A_C$ cationiques ou potentiellement cationiques,
- des unités $A_A$ anionique ou potentiellement anioniques, et
- optionnellement des unités $A_N$ neutres, hydrophiles et/ou hydrophobes,

Dans la partie A, les unités $A_C$, $A_A$ et éventuellement $A_N$ sont des préférence sous forme d'un copolymère statistique ou à gradient. Il n'est toutefois pas exclu qu'elles soient sous forme d'un copolymère légèrement bloc («blocky»).

Par unités $A_C$ cationiques ou potentiellement cationiques, on entend des unités qui comprennent un groupe cationique ou potentiellement cationique. Les unités ou groupes cationiques sont des unités ou groupes qui présentent au moins une charge positive (généralement associée à un ou plusieurs anions comme l'ion chlorure, l'ion bromure, un groupe sulfate, un groupe méthylesulfate), quel que soit le pH du milieu où est présent le copolymère. Les unités ou groupes potentiellement cationiques sont des unités ou groupes qui peuvent être neutres ou présenter au moins une charge positive selon le pH du milieu où est présent le copolymère. Dans ce cas on parlera d'unités potentiellement cationiques $A_C$ sous forme neutre ou sous forme cationique. Par extension on peut parler de monomères cationiques ou potentiellement cationiques.

Par unités $A_A$ anioniques ou potentiellement anioniques, on entend des unités qui comprennent un groupe anionique ou potentiellement anionique. Les unités ou groupes anioniques sont des unités ou groupes qui présentent au moins une charge négative (généralement associée à un ou plusieurs cations comme des cations de composés alcalins ou alcalino-terreux, par exemple le sodium, ou groupes cationiques comme l'ammonium), quel que soit le pH du milieu où est présent le copolymère. Les unités ou groupes potentiellement anioniques sont des unités ou groupe qui peuvent être neutres ou présenter au moins une charge négative selon le pH du milieu où est présent le copolymère. Dans ce cas on parlera de d'unités potentiellement anioniques $A_A$ sous forme neutre ou sous forme anionique. Par extension on peut parler de monomères anioniques ou potentiellement anioniques.

Par unités $A_N$ neutres, on entend des unités qui ne présentent pas de charge, quel que soit le pH du milieu où est présent le copolymère.

La partie A peut présenter simultanément des unités $A_C$ potentiellement cationiques sous forme cationique, et des unités $A_A$ anioniques ou potentiellement anioniques sous forme anionique. Alternativement la partie A peut présenter simultanément des unités $A_C$ cationiques ou potentiellement cationiques sous forme cationique, et des unités $A_A$ potentiellement anioniques sous forme neutre. Alternativement la partie A peut présenter simultanément des unités $A_C$ potentiellement cationiques sous forme neutre, et des unités $A_A$ anioniques ou potentiellement anioniques sous forme anionique. De préférence, le copolymère ne présente pas simultanément des unités $A_A$ et des unités $A_C$ sous forme neutre.

Les unités $A_N$ peuvent être hydrophiles ou hydrophobes. De préférence elles sont hydrophobes, mais peuvent comprendre des unités hydrophiles.

A titre d'exemples de <u>monomères hydrophiles potentiellement cationiques</u> (dont peuvent dériver des unités $A_C$), on peut mentionner :

● les N,N(dialkylamino$\omega$alkyl)amides d'acides carboxyliques $\alpha$-$\beta$ monoéthyléniquement insaturés comme le N,N-diméthylaminométhyl -acrylamide ou -méthacrylamide, le 2(N,N-diméthylamino)éthyl-acrylamide ou - méthacrylamide, le 3(N,N-diméthylamino)propyl-acrylamide ou -méthacrylamide, le 4(N,N-diméthylamino)butyl-acrylamide ou -méthacrylamide

● les aminoesters $\alpha$-$\beta$ monoéthyléniquement insaturés comme le 2(diméthyl amino)éthyl acrylate (ADAM), 2(diméthyl amino)éthyl méthacrylate (DMAM), le 3(diméthyl amino)propyl méthacrylate, le 2(tertiobutylamino)éthyl méthacrylate, le 2(dipentylamino)éthyl méthacrylate, le 2(diéthylamino)éthyl méthacrylate

● les vinylpyridines

● la vinyl amine

● les vinylimidazolines

● des monomères précurseurs de fonctions amines tels que le N-vinyl formamide, le N-vinyl acétamide, ... qui engendrent des fonctions amines primaires par simple hydrolyse acide ou basique.

A titre d'exemples de <u>monomères hydrophiles cationiques</u>, dont peuvent dériver des unités $A_C$, on peut mentionner :

● les monomères ammoniumacryloyles ou acryloyloxy comme le chlorure de triméthylammoniumpropylméthacrylate, le chlorure ou le bromure de triméthylammoniuméthylacrylamide ou méthacrylamide, le méthylsulfate de triméthylammoniumbutylacrylamide ou méthacrylamide, le méthylsulfate de trimethylammoniumpropylméthacrylamide (MES), le chlorure de (3-méthacrylamidopropyl)triméthylammonium (MAPTAC), le chlorure de (3-acrylamidopropyl) triméthylammonium (APTAC), le chlorure ou le méthylsulfate de méthacryloyloxyéthyl triméthylammonium, le chlorure d'acryloyloxyéthyl triméthylammonium ;

● le bromure, chlorure ou méthylsulfate de 1-éthyl 2-vinylpyridinium, de 1-éthyl 4-vinylpyridinium ;

● les monomères N,N-dialkyldiallylamines comme le chlorure de N,N-diméthyldiallylammonium (DADMAC) ;

● les monomères polyquaternaires comme le chlorure de diméthylaminopropylméthacrylamide,N-(3-chloro-2-hydroxypropyl) triméthylammonium (DIQUAT) ...

Des exemples de <u>monomères non-ioniques (neutres) hydrophiles ou hydrophobes,</u> dont peuvent dériver des unités $A_N$, ont déjà été mentionnés plus haut (concernant la partie B).

A titre d'exemples de <u>monomères anioniques ou potentiellement anioniques,</u> dont peuvent dériver des unités $A_A$, on peut mentionner :

● des monomères possédant au moins une fonction carboxylique, comme les acides carboxyliques $\alpha$-$\beta$ éthyléniquement insaturés ou les anhydrides correspondants, tels que les acides ou anhydrides acrylique, méthacrylique, maleique, l'acide fumarique, l'acide itaconique, le N-méthacroyl alanine, le N-acryloylglycine et leurs sels hydrosolubles

● des monomères précurseurs de fonctions carboxylates, comme l'acrylate de tertiobutyle, qui engendrent, après polymérisation, des fonctions carboxyliques par hydrolyse.

● des monomères possédant au moins une fonction sulfate ou sulfonate, comme le 2-sulfooxyethyl méthacrylate, l'acide vinylbenzène sulfonique, l'acide allyl sulfonique, le 2-acrylamido-2méthylpropane sulfonique, l'acrylate ou le méthacrylate de sulfoethyle , l'acrylate ou le méthacrylate de sulfopropyle et leurs sels hydrosolubles

● des monomères possédant au moins une fonction phosphonate ou phosphate, comme l'acide vinylphosphonique,... les esters de phosphates éthyléniquement insaturés tels que les phosphates dérivés du méthacrylate d'hydroxyéthyle (Empicryl 6835 de RHODIA) et ceux dérivés des méthacrylates de polyoxyalkylènes et leurs sels hydrosolubles

La masse molaire moyenne en nombre de la partie A, peut aller de 500 à 100000, de préférence de 500 à 25000 g/mol, mesurée par chromatographie d'exclusion stérique.

De manière particulière, la masse molaire moyenne en nombre du copolymère selon l'invention est comprise entre 1000 et 200 000 g/mol, de préférence entre 1000 et 50 000 g/mol, plus particulièrement entre 3000 et 30 000 g/mol, déterminée par GPC couplée à la méthode MALLS (Multi Angle Laser Light Scattering).

La partie A présente une charge moyenne Q positive, négative ou neutre. Dans le cas d'une partie A amphotère, on peut moduler les propriétés du polymère, et ses utilisations, en variant la charge moyenne. Dans un mode de réalisation particulier, la partie B est une partie neutre, et en ce que la partie B présente une charge moyenne Q positive, négative ou neutre.

A titre d'exemples de copolymères diblocs, on peut mentionner notamment les copolymères (polyacrylate de butyle) - (poly(acide acrylique-stat-acrylate de 2-diméthylaminoéthyle quaternisé)). A titre d'exemple de polymères triblocs, on peut mentionner notamment les copolymères (poly(acide acrylique-stat-acrylate de 2-diméthylaminoéthyle quaternisé))-polyorganosiloxanes-(poly(acide acrylique-stat-acrylate de 2-diméthylaminoéthyle quaternisé)).

Les copolymères selon l'invention peuvent être obtenus par toute méthode connue, que ce soit par polymérisation radicalaire, contrôlée ou non, par polymérisation par ouverture de cycle (notamment anionique ou cationique), par polymérisation anionique ou cationique, ou encore par modification chimique d'un polymère.
De manière préférée, on met en oeuvre des méthodes de polymérisation radicalaire dite vivante ou contrôlée.
A titre d'exemple de procédés de polymérisation dite vivante ou contrôlée, on peut notamment se référer à :

- les procédés des demandes WO 98/58974, WO 00/75207 et WO 01/42312 qui mettent en oeuvre une polymérisation radicalaire contrôlée par des agents de contrôle de type xanthates,
- le procédé de polymérisation radicalaire contrôlée par des agents de contrôles de type dithioesters de la demande WO 98/01478,
- le procédé décrit dans la demande WO 02/08307, notamment pour l'obtention de copolymères comprenant des bloc polyorganosiloxane,
- le procédé de polymérisation radicalaire contrôlée par des agents de contrôle de type dithiocarbamates de la demande WO 99/31144,
- le procédé de polymérisation radicalaire contrôlée par des agents de contrôle de type dithiocarbazates de la demande WO 02/26836,
- le procédé de polymérisation radicalaire contrôlée par des agents de contrôle de type dithiophosphoroesters de la demande WO 02/10223,
  (éventuellement les copolymères à blocs obtenus comme ci-dessus par polymérisation radicalaire contrôlée, peuvent subir une réaction de purification de leur extrémité de chaîne soufrée, par exemple par des procédés de type hydrolyse, oxydation, réduction, pyrolyse ou substitution)
- le procédé de la demande WO 99/03894 qui met en oeuvre une polymérisation en présence de précurseurs nitroxydes,
- le procédé de la demande WO 96/30421 qui utilise une polymérisation radicalaire par transfert d'atome (ATRP),
- le procédé de polymérisation radicalaire contrôlée par des agents de contrôle de type iniferters selon l'enseignement de Otu et al., Makromol. Chem. Rapid. Commun., 3, 127 (1982),
- le procédé de polymérisation radicalaire contrôlée par transfert dégénératif d'iode selon l'enseignement de Tatemoto et al., Jap. 50, 127, 991 (1975), Daikin Kogyo Co ltd Japan et Matyjaszewski et al., Macromolecules, 28, 2093 (1995),
- le procédé de polymérisation radicalaire contrôlée par les dérivés du tetraphényléthane, divulgué par D. Braun et al. Dans Macromol. Symp. 111,63 (1996), ou encore,
- le procédé de polymérisation radicalaire contrôlée par des complexes organocobalt décrit par Wayland et al. Dans J.Am.Chem.Soc. 116,7973 (1994)
- le procédé de polymérisation radicalaire contrôlée par du diphénylethylène (WO 00/39169 ou WO 00/37507).

Lorsqu'il s'agit de copolymères à architecture contrôlée greffés ou peignes, ceux-ci peuvent être obtenus par des méthodes dites de greffage direct et copolymérisation. Le greffage direct consiste à polymériser le(s) monomère (s) choisi(s) par voie radicalaire, en présence du polymère sélectionné pour former le squelette du produit final. Si le couple monomère / squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.
Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".
Le greffage peut être réalisé avantageusement en présence d'un agent de contrôle de la polymérisation tel que cité dans les références ci-dessus.

Les procédés de préparation de polymères en forme d'étoile peuvent être essentiellement classés en deux groupes. Le premier correspond à la formation des bras des polymères à partir d'un composé plurifonctionnel constituant le centre (technique "core-first") (Kennedy, J.P. and coll. Macromolecules, 29, 8631 (1996), Deffieux, A. and coll. Ibid, 25, 6744, (1992), Gnanou, Y. and coli. Ibid, 31, 6748 (1998)) et le second correspond à une méthode où les molécules de polymères qui vont constituer les bras sont d'abord synthétisées et ensuite liées ensemble sur un coeur pour former un polymère en forme d'étoile (technique "arm-first").

A titre d'exemple de synthèse de ce type de polymère, on pourra se référer au brevet WO 00/02939. On peut citer également les procédés de polymérisation à partir d'un coeur comprenant plusieurs groupes de transfert, et les procédés de réticulation de micelles.

D'autres détails ou avantages de l'invention apparaîtront plus clairement au vu de l'exemple qui suit, sans caractère limitatif.

Les abréviations données ont la signification

* P(ABu)                Homopolymère de l'acrylate de butyle (ABu)
* P(ADAME)              Bloc homopolymère du diméthylaminoéthyl acrylate(ADAME)
* P(ADAMQuat)           Bloc homopolymère du méthyl sulfate de triméthylammoniuméthyl acrylate (ADAMQuat)
* P(AAstatADAMQuat)     Bloc copolymère statistique de l'acide acrylique et de l'ADAMQuat de-rapport pondéral AA/ADAMQuat de 30/70

**Synthèse d'un copolymère dibloc Polyacrylate de butyle Poly(acide acrylique-stat-acrylate de 2-diméthylaminoéthyle quaternisé)**

Dans un ballon bicol en verre de 500 ml muni d'un réfrigérant et d'une agitation magnétique et maintenu sous argon, on introduit 124,2 g d'éthanol, 13,54 g de O-ethyl-S-(1-methoxycarbonyl)ethylenyl) xanthate $(CH_3CHCO_2CH_3)S$ $(C=S)OEt$ et 65 g d'acrylate de butyle. La solution est mise en température à 70°C, et 4,27 g d'azobisisobutyronitrile (AIBN) sont ajoutés au mélange réactionnel. La réaction est maintenue pendant deux heures à cette température. Une analyse RMN1H confirme que le monomère acrylate a été totalement polymérisé. La masse molaire du polymère est mesurée par chromatographie d'exclusion stérique. Mn=1800 g/mol.

Au polymère issu de la première étape, maintenu à 70°C, sont ajoutés 2,13 g d'azobisisobutyronitrile. Ensuite, un mélange contenant 173,3 g d'acide acrylique, 404,7 g d' acrylate de 2-diméthylaminoéthyl quaternisé, 335 g d'eau et 335 g d'éthanol est additionné pendant 4 heures. Au bout de 2 heures d'introduction, 3,2 g d'AIBN sont introduits. A la fin des 4 heures d'introduction de la solution de monomères, 3,2 g d'AIBN sont à nouveau introduits dans le réacteur. Le réaction est ensuite maintenue à cette température pendant 2 heures supplémentaires.

Une analyse RMN 1 H confirme que la composition du copolymère final correspond à celle attendue.

**Revendications**

1. Copolymère à structure contrôlée, comprenant au moins deux parties A et B de compositions distinctes, la partie A comprenant des unités ioniques ou potentiellement ioniques, **caractérisé en ce que**:

   | - la partie A est une partie amphotère comprenant:

   - des unités $A_C$ cationiques ou potentiellement cationiques,
   - des unités $A_A$ anioniques ou potentiellement anioniques, et
   - optionnellement des unités $A_N$ neutres, hydrophiles et/ou hydrophobes,

   - la partie B n'est pas une partie amphotère ou zwitterionique.

2. Copolymère selon la revendication précédente, **caractérisé en ce que** la partie B est une partie neutre, hydrophile ou hydrophobe, comprenant des unités neutres, hydrophiles ou hydrophobes.

3. Copolymère selon la revendication 1 ou 2, **caractérisé en ce qu'**il a l'une des structures suivantes:

   - copolymère à blocs, comprenant au moins deux blocs, la partie A correspondant à un bloc, la partie B correspondant à un autre, la partie A présentant éventuellement un gradient de composition,
   - copolymère peigne ou greffé, comprenant un squelette et des chaînes latérales, avec la partie A correspondant au squelette et la partie B correspondant à des chaînes latérales, ou avec la partie B correspondant au squelette et la partie A correspondant à des chaînes latérales,
   - copolymère étoile ou microgel, comprenant un coeur polymérique ou non polymérique, et des chaînes polymériques périphériques, une partie correspondant au coeur, l'autre correspondant aux chaînes périphériques.

**4.** Copolymère selon l'une des revendications précédentes, **caractérisé en ce que** c'est un copolymère dibloc (bloc A)-(bloc B), triblock (bloc A)-(bloc B)-(bloc A) ou tribloc (bloc B)-(bloc A)-(bloc B)

**5.** Copolymère selon l'une des revendications précédentes, **caractérisé en ce qu'**il est obtenu par un procédé de polymérisation radicalaire contrôlée.

**6.** Copolymère selon l'une des revendications précédentes, **caractérisé en ce que** la partie B est une partie neutre, et **en ce que** la partie A présente une charge moyenne Q positive, négative ou neutre.

**7.** Copolymère selon l'une des revendications précédentes, **caractérisé en ce qu'**il est sous forme de poudre, sous forme de dispersion dans un liquide ou sous forme de solution dans un solvant.

**8.** Copolymère selon l'une des revendications précédentes, **caractérisé en ce que** les unités $A_C$ dérivent des monomères sont sélectionnés parmi:

● les N,N(dialkylaminoωalkyl)amides d'acides carboxyliques α-β monoéthyléniquement insaturés comme le N,N-diméthylaminométhyl -acrylamide ou -méthacrylamide, le 2(N,N-diméthylamino)éthyl-acrylamide ou-méthacrylamide, le 3(N,N-diméthylamino)propyl-acrylamide ou -méthacrylamide, le 4(N,N-diméthylamino)butyl-acrylamide ou -méthacrylamide

● les aminoesters α-β monoéthyléniquement insaturés comme le 2(diméthyl amino)éthyl acrylate (ADAM), 2(diméthyl amino)éthyl méthacrylate (DMAM), le 3(diméthyl amino)propyl méthacrylate, le 2(tertiobutylamino) éthyl méthacrylate, le 2(dipentylamino)éthyl méthacrylate, le 2(diéthylamino)éthyl méthacrylate

● les vinylpyridines

● la vinyl amine

● les vinylimidazolines

● des monomères précurseurs de fonctions amines tels que le N-vinyl formamide, le N-vinyl acétamide, qui engendrent des fonctions amines primaires par simple hydrolyse acide ou basique,

● les monomères ammoniumacryloyles ou acryloyloxy comme le chlorure de triméthylammoniumpropylméthacrylate, le chlorure ou le bromure de triméthylammoniuméthylacrylamide ou méthacrylamide, le méthylsulfate de triméthylammoniumbutylacrylamide ou méthacrylamide, le méthylsulfate de triméthylammoniumpropylméthacrylamide (MES), le chlorure de (3-méthacrylamidopropyl)triméthylammonium (MAPTAC), le chlorure de (3-acrylamidopropyl)triméthylammonium (APTAC), le chlorure ou le méthylsulfate de méthacryloytoxyéthyl triméthylammonium, le chlorure d'acryloyloxyéthyl triméthylammonium ;

● le bromure, chlorure ou méthylsulfate de 1-éthyl 2-vinylpyridinium, de 1-éthyl 4-vinylpyridinium;

● les monomères N,N-dialkyldiallylamines comme le chlorure de N,N-diméthyldiallylammonium (DADMAC) ;

● les monomères polyquaternaires comme le chlorure de diméthylaminopropylméthacrylamide, N-(3-chloro-2-hydroxypropyl) triméthylammonium (DIQUAT).

**9.** Copolymère selon l'une des revendications précédentes, **caractérisé en ce que** les unités $A_A$ dérivent des monomères sont sélectionnés parmi:

● des monomères possédant au moins une fonction carboxylique, comme les acides carboxyliques α-β éthyléniquement insaturés ou les anhydrides correspondants, tels que les acides ou anhydrides acrylique, méthacrylique, maleique, l'acide fumarique, l'acide itaconique, le N-méthacroyl alanine, le N-acryloylglycine et leurs sels hydrosolubles

● des monomères précurseurs de fonctions carboxylates, comme l'acrylate de tertiobutyle, qui engendrent, après polymérisation, des fonctions carboxyliques par hydrolyse

● des monomères possédant au moins une fonction sulfate ou sulfonate, comme le 2-sulfooxyethyl méthacrylate, l'acide vinylbenzène sulfonique, l'acide allyl sulfonique, le 2-acrylamido-2méthylpropane sulfonique, l'acrylate ou le méthacrylate de sulfoethyle, acrylate ou le méthacrylate de sulfopropyle et leurs sels hydrosolubles

● des monomères possédant au moins une fonction phosphonate ou phosphate, comme l'acide vinylphosphonique, les esters de phosphates éthyléniquement insaturés tels que les phosphates dérivés du méthacrylate d'hydroxyéthyle et ceux dérivés des méthacrylates de polyoxyalkylènes et leurs sels hydrosolubles.

**10.** Copolymère selon l'une des revendications précédentes, **caractérisé en ce que** les unités $A_N$ dérivent des monomères sont sélectionnés parmi:

● les monomères vinylaromatiques tels que styrène, alpha-méthylstyrène, vinyltoluène

● les halogénures de vinyle ou de vinylidène, comme le chlorure de vinyle, chlorure de vinylidène

● les C$_1$-C$_{12}$ alkylesters d'acides α-β monoéthylsniquement insaturés tels que les acrylates et méthacrylates de méthyle, éthyle, butyle, acrylate de 2-éthylhexyle

● les esters de vinyle ou d'allyle d'acides carboxyliques saturés tels que les acétates, propionates, versatates, stéarates de vinyle ou d'allyle

● les nitriles α-β monoéthyléniquement insaturés contenant de 3 à 12 atomes de carbone, comme l'acrylonitrile, le methacrylonitrile

● les α-oléfines comme l'éthylène

● les diènes conjugués, comme le butadiène, l'isoprène, le chloroprène,

● les monomères susceptibles des générer des chaînes polydiméthylsiloxane (PDMS),

● les hydroxyalkylesters d'acides α-β éthyléniquement insaturés comme les acrylates et méthacrylates d'hydroxyéthyle, d'hydroxypropyle, le glycérol monométhacrylate

● les amides α-β éthyléniquement insaturés comme l'acrylamide, le N,N-diméthyl méthacrylamide, le N-méthylolacrytamide

● les monomères α-β éthyléniquement insaturés portant un segment polyoxyalkyléné hydrosoluble du type polyoxyde d'éthylène, comme les polyoxyde d'éthylène α-méthacrylates ou α,ω-diméthacrylates, le méthacrylates de polyoxyéthylène ω-béhényle, le méthacrylate de polyoxyéthylène ω-tristyrylphényle)

● les monomères α-β éthyléniquement insaturés précurseurs d'unités ou de segments hydrophiles tels que l'acétate de vinyle qui, une fois polymérisé, peut être hydrolysé pour engendrer des unités alcool vinylique ou des segments alcool polyvinylique

● les vinylpyrrolidones

● les monomères α-β éthyléniquement insaturés de type uréido et en particulier le méthacrylamido de 2-imidazolidinone éthyle.

**11.** Copolymère selon l'une des revendications précédentes, **caractérisé en ce que** la partie B comprend des unités dérivant de monomères neutres hydrophobes sélectionnés parmi:

● les monomères vinylaromatiques tels que styrène, alpha-méthylstyrène, vinyltoluène,

● les halogénures de vinyle ou de vinylidène, comme le chlorure de vinyle, chlorure de vinylidène,

● les C$_1$-C$_{12}$ alkylesters d'acides α-β monoéthyléniquement insaturés tels que les acrylates et méthacrylates de méthyle, éthyle, butyle, acrylate de 2-éthylhexyle,

● les esters de vinyle ou d'allyle d'acides carboxyliques saturés tels que les acétates, propionates, versatates, stéarates de vinyle ou d'allyle,

● les nitriles α-β monoéthyléniquement insaturés contenant de 3 à 12 atomes de carbone, comme l'acrylonitrile, le methacrylonitrile,

● les α-oléfines comme l'éthylène,

● les diènes conjugués, comme le butadiène, l'isoprène, le chloroprène

● les monomères susceptibles de générer des chaînes polydiméthylsiloxane.

**12.** Copolymère selon l'une des revendications 3 à 11, **caractérisé en ce que en ce qu'**il a une structure copolymère à blocs, comprenant au moins deux blocs, la partie A correspondant à un bloc, la partie B correspondant à un autre, la partie A présentant une structure copolymère statistique ou copolymére à gradient de composition.

**13.** Utilisation d'un copolymère selon l'une des revendications précédentes, dans des compositions de détergence, de soin du linge, ou de nettoyage, traitement, et/ou protection de la peau et/ou des cheveux.

## Claims

**1.** Controlled structure copolymer comprising at least two parts A and B of different compositions, part A comprising ionic or potentially ionic units, **characterized in that**:

- part A is an amphoteric part, comprising:

- cationic or potentially cationic units A$_C$,
- anionic or potentially anionic units A$_A$, and
- optionally, hydrophilic and/or hydrophobic neutral units A$_N$,

- part B is not an amphoteric or zwitterionic part.

2. Copolymer according to the preceding claim, **characterized in that** part B is a hydrophilic or hydrophobic neutral part comprising hydrophilic or hydrophobic neutral units.

3. Copolymer according to Claim 1 or 2, **characterized in that** it has one of the following structures:

   - block copolymer, comprising at least two blocks, part A corresponding to one block, part B corresponding to another, part A optionally having a composition gradient,
   - comb or grafted copolymer, comprising a backbone and side chains, with part A corresponding to the backbone and part B corresponding to side chains, or with part B corresponding to the backbone and part A corresponding to side chains,
   - star or microgel copolymer, comprising a polymeric or nonpolymeric core, and peripheral polymeric chains, one part corresponding to the core, the other corresponding to the peripheral chains.

4. Copolymer according to one of the preceding claims, **characterized in that** it is a (block A)-(block B) diblock copolymer, a (block A)-(block B)-(block A) triblock copolymer or a (block B)-(block A)-(block B) triblock copolymer.

5. Copolymer according to one of the preceding claims, **characterized in that** it is obtained by means of a controlled radical polymerization process.

6. Copolymer according to one of the preceding claims, **characterized in that** part B is a neutral part, and **in that** part A has a positive, negative or neutral average charge Q.

7. Copolymer according to one of the preceding claims, **characterized in that** it is in the form of a powder, in the form of a dispersion in a liquid, or in the form of a solution in a solvent.

8. Copolymer according to one of the preceding claims, **characterized in that** the units $A_C$ derived from the monomers are selected from:

   ● N,N-(dialkylamino-$\omega$-alkyl)amides of $\alpha,\beta$-monoethylenically unsaturated carboxylic acids, such as N,N-dimethylaminomethylacrylamide or -methacrylamide, 2-(N,N-dimethylamino)ethylacrylamide or -methacrylamide, 3-(N,N-dimethylamino)propylacrylamide or -methacrylamide, and 4-(N,N-dimethylamino)butylacrylamide or -methacrylamide,
   ● $\alpha,\beta$-monoethylenically unsaturated amino esters such as 2-(dimethylamino)ethyl acrylate (DMAA), 2-(dimethylamino)ethyl methacrylate (DMAM), 3-(dimethylamino)propyl methacrylate, 2-(tert-butyl-amino)ethyl methacrylate, 2-(dipentylamino)ethyl methacrylate, and 2(diethylamino)ethyl methacrylate,
   ● vinylpyridines,
   ● vinylamine,
   ● vinylimidazolines,
   ● monomers that are precursors of amine functions such as N-vinylformamide, N-vinylacetamide, which give rise to primary amine functions by simple acid or base hydrolysis,
   ● acryloyl- or acryloyloxyammonium monomers such as trimethylammonium propyl methacrylate chloride, trimethylammonium ethylacrylamide or -methacrylamide chloride or bromide, trimethylammonium butylacrylamide or -methacrylamide methyl sulfate, trimethylammonium propylmethacrylamide methyl sulfate (MES), (3-methacrylamidopropyl)trimethylammonium chloride (MAPTAC), (3-acrylamidopropyl)trimethylammonium chloride (APTAC), methacryloyloxyethyl-trimethylammonium chloride or methyl sulfate, and acryloyloxyethyltrimethylammonium chloride;
   ● 1-ethyl-2-vinylpyridinium or 1-ethyl-4-vinylpyridinium bromide, chloride or methyl sulfate;
   ● N,N-dialkyldiallylamine monomers such as N,N-di-methyldiallylammonium chloride (DADMAC);
   ● polyquaternary monomers such as dimethylamino-propylmethacrylamide chloride and N-(3-chloro-2-hydroxypropyl)trimethylammonium (DIQUAT).

9. Copolymer according to one of the preceding claims, **characterized in that** the units $A_A$ derived from the monomers are selected from:

   ● monomers having at least one carboxylic function, for instance $\alpha,\beta$-ethylenically unsaturated carboxylic acids or the corresponding anhydrides, such as acrylic, methacrylic or maleic acids or anhydrides, fumaric acid, itaconic acid, N-methacroylalanine, N-acryloylglycine, and their water-soluble salts,

● monomers that are precursors of carboxylate functions, such as tert-butyl acrylate, which, after polymerization, give rise to carboxylic functions by hydrolysis,

● monomers having at least one sulfate or sulfonate function, such as 2-sulfooxyethyl methacrylate, vinylbenzene sulfonic acid, allyl sulfonic acid, 2-acrylamido-2-methylpropane sulfonic acid, sulfoethyl acrylate or methacrylate, sulfopropyl acrylate or methacrylate, and their water-soluble salts,

● monomers having at least one phosphonate or phosphate function, such as vinylphosphonic acid, the esters of ethylenically unsaturated phosphates, such as the phosphates derived from hydroxyethyl methacrylate and those derived from polyoxyalkylene methacrylates, and their water-soluble salts.

10. Copolymer according to one of the preceding claims, **characterized in that** the units $A_N$ derived from the monomers are selected from:

● vinylaromatic monomers such as styrene, alphamethylstyrene, vinyltoluene,

● vinyl halides or vinylidene halides, such as vinyl chloride, vinylidene chloride,

● $C_1$-$C_{12}$ alkylesters of $\alpha,\beta$-monoethylenically unsaturated acids such as methyl, ethyl or butyl acrylates and methacrylates, 2-ethylhexyl acrylate,

● vinyl esters or allyl esters of saturated carboxylic acids, such as vinyl or allyl acetates, propionates, versatates, stearates,

● $\alpha,\beta$-monoethylenically unsaturated nitriles containing from 3 to 12 carbon atoms, such as acrylonitrile, methacrylonitrile,

● $\alpha$-olefins such as ethylene,

● conjugated dienes, such as butadiene, isoprene, chloroprene,

● monomers capable of generating polydimethylsiloxane (PDMS) chains,

● hydroxyalkyl esters of $\alpha,\beta$-ethylenically unsaturated acids, such as hydroxyethyl or hydroxypropyl acrylates and methacrylates, glyceryl monomethacrylate,

● $\alpha,\beta$-ethylenically unsaturated amides such as acrylamide, N,N-dimethylmethacrylamide, N-methylolacrylamide,

● $\alpha,\beta$-ethylenically unsaturated monomers bearing a water-soluble polyoxyalkylene segment of the polyethylene oxide type, such as polyethylene oxide $\alpha$-methacrylates or $\alpha,\omega$-dimethacrylates, $\omega$-behenyl polyoxyethylene methacrylate, and $\omega$-tristyrylphenyl polyoxyethylene methacrylate,

● $\alpha,\beta$-ethylenically unsaturated monomers which are precursors of hydrophilic units or segments, such as vinyl acetate, which, once polymerized, can be hydrolyzed in order to give rise to vinyl alcohol units or polyvinyl alcohol segments,

● vinylpyrrolidones,

● $\alpha,\beta$-ethylenically unsaturated monomers of the ureido type, and in particular 2-imidazolidinone-ethyl methacrylamide.

11. Copolymer according to one of the preceding claims, **characterized in that** part B comprises units derived from hydrophobic neutral monomers selected from:

● vinylaromatic monomers such as styrene, alphamethylstyrene, vinyltoluene,

● vinyl halides or vinylidene halides, such as vinyl chloride, vinylidene chloride,

● $C_1$-$C_{12}$ alkylesters of $\alpha,\beta$-monoethylenically unsaturated acids such as methyl, ethyl or butyl acrylates and methacrylates, 2-ethylhexyl acrylate,

● vinyl esters or allyl esters of saturated carboxylic acids, such as vinyl or allyl acetates, propionates, versatates, stearates,

● $\alpha,\beta$-monoethylenically unsaturated nitriles containing from 3 to 12 carbon atoms, such as acrylonitrile, methacrylonitrile,

● $\alpha$-olefins such as ethylene,

● conjugated dienes, such as butadiene, isoprene, chloroprene,

● monomers capable of generating polydimethylsiloxane chains.

12. Copolymer according to one of Claims 3 to 11, **characterized in that** it has a block copolymer structure, comprising at least two blocks, part A corresponding to one block, part B corresponding to another, part A having a random copolymer or composition gradient copolymer structure.

13. Use of a copolymer according to one of the preceding claims, in detergent compositions, fabric care compositions, or compositions for cleansing, treating and/or protecting the skin and/or the hair.

**Patentansprüche**

1. Copolymer mit kontrollierter Struktur, umfassend mindestens zwei Teile A und B unterschiedlicher Zusammensetzung, wobei der Teil A ionische oder potenziell ionische Einheiten umfasst, **dadurch gekennzeichnet, dass**:

   - der Teil A ein amphoterer Teil ist, der Folgendes umfasst:

     - kationische oder potenziell kationische Einheiten $A_C$,
     - anionische oder potenziell anionische Einheiten $A_A$ und
     - gegebenenfalls hydrophile und/oder hydrophobe neutrale Einheiten $A_N$,

   - der Teil B kein amphoterer oder zwitterionischer Teil ist.

2. Copolymer nach dem vorhergehenden Anspruch, **dadurch gekennzeichnet, dass** der Teil B ein hydrophiler oder hydrophober neutraler Teil, der hydrophile oder hydrophobe neutrale Einheiten umfasst, ist.

3. Copolymer nach Anspruch 1 oder 2, **dadurch gekennzeichnet, dass** es eine der folgenden Strukturen aufweist:

   - Blockcopolymer mit mindestens zwei Blöcken, wobei der Teil A einem Block entspricht, der Teil B einem anderen Block entspricht und der Teil A gegebenenfalls einen Zusammensetzungsgradienten aufweist,
   - Kamm- oder Pfropfcopolymer mit einer Hauptkette und Seitenketten, wobei der Teil A der Hauptkette entspricht und der Teil B den Seitenketten entspricht oder der Teil B der Hauptkette entspricht und der Teil A den Seitenketten entspricht,
   - Stern- oder Mikrogelcopolymer mit einem polymeren oder nichtpolymeren Kern und peripheren Polymerketten, wobei ein Teil dem Kern entspricht und der andere den peripheren Ketten entspricht.

4. Copolymer nach einem der vorhergehenden Ansprüche, **dadurch gekennzeichnet, dass** es sich um ein (Block A)-(Block-B)-Diblockcopolymer, (Block A)-(Block-B)-(Block A)-Triblockcopolymer oder (Block B)-(Block-A)-(Block B)-Triblockcopolymer handelt.

5. Copolymer nach einem der vorhergehenden Ansprüche, **dadurch gekennzeichnet, dass** es durch ein Verfahren zur kontrollierten radikalischen Polymerisation erhalten wird.

6. Copolymer nach einem der vorhergehenden Ansprüche, **dadurch gekennzeichnet, dass** Teil B ein neutraler Teil ist und Teil A eine positive, negative oder neutrale durchschnittliche Ladung Q aufweist.

7. Copolymer nach einem der vorhergehenden Ansprüche, **dadurch gekennzeichnet, dass** es in Form eines Pulvers, in Form einer Dispersion in einer Flüssigkeit oder in Form einer Lösung in einem Lösungsmittel vorliegt.

8. Copolymer nach einem der vorhergehenden Ansprüche, **dadurch gekennzeichnet, dass** sich die Einheiten $A_C$ von Monomeren ableiten, die ausgewählt sind aus:

   ● N,N-(Dialkylamino-$\omega$-alkyl)amiden von $\alpha,\beta$-monoethylenisch ungesättigten Carbonsäuren wie N,N-Dimethylaminomethylacrylamid oder -methacrylamid, 2-(N,N-Dimethylamino)ethylacrylamid oder -methacrylamid, 3-(N,N-Dimethylamino)-propylacrylamid oder -methacrylamid, 4-(N,N-Dimethylamino)butylacrylamid oder -methacrylamid,
   ● a,$\beta$-monoethylenisch ungesättigten Aminoestern wie 2-(Dimethylamino)ethylacrylat (ADAM), 2-(Dimethylamino)ethylmethacrylat (DMAM), 3-(Dimethylamino)propylmethacrylat, 2-(tert.-Butylamino)ethylmethacrylat, 2-(Dipentylamino)-ethylmethacrylat, 2-(Diethylamino)ethylmethacrylat,
   ● Vinylpyridinen,
   ● Vinylamin,
   ● Vinylimidazolinen,
   ● Monomeren, die Vorläufer von Aminfunktionen sind, wie N-Vinylformamid und N-Vinylacetamid, die durch einfache saure oder basische Hydrolyse Aminfunktionen ergeben,
   ● Acryloyl- oder Acryloyloxyammonium-Monomeren wie Trimethylammoniumpropylmethacrylatchlorid, Trimethylammoniumethylacrylamidchlorid oder -bromid oder Trimethylammoniummethacrylamidchlorid oder -bromid, Trimethylammoniumbutylacrylamidmethylsulfat oder Trimethylammoniumbutylmethacrylamidmethylsulfat, Trimethylammoniumpropylmethacrylamidmethylsulfat (MES), (3-Methacrylamidopropyl)trimethylammoniumchlorid (MAPTAC), (3-Acrylamidopropyl)trimethylammoniumchlorid (APTAC), (Methacryloyl-oxyethyl)trimethylammoniumchlorid oder -methylsulfat, (Acryloyloxyethyl)trimethylammoniumchlorid,

● 1-Ethyl-2-vinylpyridiniumbromid, -chlorid oder -methylsulfat, 1-Ethyl-4-vinylpyridiniumbromid, -chlorid oder -methylsulfat,

● N,N-Dialkyldiallylamin-Monomeren wie N,N-Dimethyldiallylammoniumchlorid (DADMAC);

● polyquaternären Monomeren wie Dimethylaminopropylmethacrylamid-N-(3-chlor-2-hydroxypropyl)trimethylammoniumchlorid (DIQUAT).

9. Copolymer nach einem der vorhergehenden Ansprüche, **dadurch gekennzeichnet, dass** sich die Einheiten $A_A$ von Monomeren ableiten, die ausgewählt sind aus:

● Monomeren mit mindestens einer Carboxylfunktion, wie $\alpha,\beta$-ethylenisch ungesättigten Carbonsäuren oder den entsprechenden Anhydriden, wie Acrylsäure, Methacrylsäure und Maleinsäure und deren Anhydriden, Fumarsäure, Itaconsäure, N-Methacroylalanin, N-Acryloylglycin und deren wasserlöslichen Salzen,

● Monomeren, die Vorläufer von Carboxylfunktionen sind, wie tert.-Butylacrylat, die nach der Polymerisation durch Hydrolyse Carboxylfunktionen ergeben,

● Monomeren mit mindestens einer Sulfat- oder Sulfonatfunktion, wie 2-Sulfooxyethylmethacrylat, Vinylbenzolsulfonsäure, Allylsulfonsäure, 2-Acrylamido-2-methylpropansulfonsäure, Sulfoethylacrylat oder -methacrylat, Sulfopropylacrylat oder -methacrylat und wasserlöslichen Salzen davon,

● Monomeren mit mindestens einer Phosphonat- oder Phosphatfunktion, wie Vinylphosphonsäure, ethylenisch ungesättigten Phosphatestern wie Phosphaten, die sich von Hydroxyethylmethacrylat ableiten, und Phosphaten, die sich von Polyoxyalkylenmethacrylaten ableiten, und deren wasserlöslichen Salzen.

10. Copolymer nach einem der vorhergehenden Ansprüche, **dadurch gekennzeichnet, dass** sich die Einheiten $A_N$ von Monomeren ableiten, die ausgewählt sind aus:

● vinylaromatischen Monomeren wie Styrol, alpha-Methylstyrol, Vinyltoluol,

● Vinyl- oder Vinylidenhalogeniden wie Vinylchlorid und Vinylidenchlorid,

● $C_1$-$C_{12}$-Alkylestern von $\alpha,\beta$-monoethylenisch ungesättigten Säuren wie Methyl-, Ethyl- und Butylacrylat und -methacrylat und 2-Ethylhexylacrylat,

● Vinyl- oder Allylestern von gesättigten Carbonsäuren wie Vinyl- oder Allylacetat, -propionat, -versatat und -stearat,

● $\alpha,\beta$-monoethylenisch ungesättigten Nitrilen mit 3 bis 12 Kohlenstoffatomen, wie Acrylnitril und Methacrylnitril,

● $\alpha$-Olefinen wie Ethylen,

● konjugierten Dienen, wie Butadien, Isopren und Chloropren,

● Monomeren, die zur Bildung von Polydimethylsiloxanketten (PDMS-Ketten) befähigt sind,

● Hydroxyalkylestern von $\alpha,\beta$-ethylenisch ungesättigten Säuren wie Hydroxyethyl- und Hydroxypropylacrylat und -methacrylat und Glycerinmonomethacrylat,

● $\alpha,\beta$-ethylenisch ungesättigten Amiden wie Acrylamid, N,N-Dimethylmethacrylamid und N-Methylolacrylamid,

● $\alpha,\beta$-ethylenisch ungesättigten Monomeren mit einem wasserlöslichen Polyoxyalkylensegment vom Polyethylenoxid-Typ, wie Polyethylenoxid-$\alpha$-methacrylaten oder -$\alpha,\omega$-dimethacrylaten, $\omega$-Behenylpolyoxyethylenmethacrylat und $\omega$-Tristyrylphenylpolyoxyethylenmethacrylat,

● $\alpha,\beta$-ethylenisch ungesättigten Monomeren, die Vorläufer von hydrophilen Einheiten oder Segmenten sind, wie Vinylacetat, das nach erfolgter Polymerisation zur Bildung von Vinylalkoholeinheiten oder Polyvinylalkoholsegmenten hydrolysiert werden kann,

● Vinylpyrrolidonen,

● $\alpha,\beta$-ethylenisch ungesättigten Monomeren vom Ureido-Typ und insbesondere 2-Imidazolidinonethylmethacrylamid.

11. Copolymer nach einem der vorhergehenden Ansprüche, **dadurch gekennzeichnet, dass** der Teil B Einheiten umfasst, die sich von hydrophoben neutralen Monomeren ableiten, die ausgewählt sind aus:

● vinylaromatischen Monomeren wie Styrol, alpha-Methylstyrol, Vinyltoluol,

● Vinyl- oder Vinylidenhalogeniden wie Vinylchlorid und Vinylidenchlorid,

● $C_1$-$C_{12}$-Alkylestern von $\alpha,\beta$-monoethylenisch ungesättigten Säuren wie Methyl-, Ethyl- und Butylacrylat und -methacrylat und 2-Ethylhexylacrylat,
● Vinyl- oder Allylestern von gesättigten Carbonsäuren wie Vinyl- oder Allylacetat, -propionat, -versatat und -stearat,
● α,β-monoethylenisch ungesättigten Nitrilen mit 3 bis 12 Kohlenstoffatomen, wie Acrylnitril und Methacrylnitril,
● α-Olefinen wie Ethylen,
● konjugierten Dienen, wie Butadien, Isopren und Chloropren,
● Monomeren, die zur Bildung von Polydimethylsiloxanketten befähigt sind.

12. Copolymer nach einem der Ansprüche 3 bis 11, **dadurch gekennzeichnet, dass** es eine Blockcopolymerstruktur mit mindestens zwei Blöcken aufweist, wobei der Teil A einem Block entspricht, der Teil B einem anderen Block entspricht und der Teil A eine statistische aufgebaute Copolymerstruktur oder eine Copolymerstruktur mit einem Zusammensetzungsgradienten aufweist.

13. Verwendung eines Copolymers nach einem der vorhergehenden Ansprüche in Waschmittelzusammensetzungen, Wäschepflegezusammensetzungen oder Zusammensetzungen zur Reinigung, zur Behandlung und/oder zum Schutz der Haut und/oder der Haare.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 0228929 A
- WO 9858974 A
- WO 0075207 A
- WO 0142312 A
- WO 9801478 A
- WO 0208307 A
- WO 9931144 A
- WO 0226836 A
- WO 0210223 A
- WO 9903894 A
- WO 9630421 A
- WO 0039169 A
- WO 0037507 A
- WO 0002939 A

### Littérature non-brevet citée dans la description

- **OTU et al.** *Makromol. Chem. Rapid. Commun.,* 1982, vol. 3, 127
- **TATEMOTO et al.** Jap. Daikin Kogyo Co ltd Japan, 1975, vol. 50, 991
- **MATYJASZEWSKI et al.** *Macromolecules,* 1995, vol. 28, 2093
- **D. BRAUN et al.** *Macromol. Symp.,* 1996, vol. 111, 63
- **WAYLAND et al.** *J.Am.Chem.Soc.,* 1994, vol. 116, 7973
- **KENNEDY, J.P.** *Macromolecules,* 1996, vol. 29, 8631
- **DEFFIEUX, A.** *MACROMOLECULES,* 1992, vol. 25, 6744
- **GNANOU, Y.** *MACROMOLECULES,* 1998, vol. 31, 6748